# EUROPÄISCHE PATENTSCHRIFT

(11) **EP 0 932 625 B1**
(45) Veröffentlichungstag und Bekanntmachung des Hinweises auf die Patenterteilung: **27.08.2003**
(21) Anmeldenummer: 97912204.1
(22) Anmeldetag: 20.10.1997
(51) Int. Cl.: C07K 16/10, C12P 21/08, G01N 33/577

(54) **MONOKLONALE ANTIKÖRPER GEGEN DAS EPITOP YPYDVPDYA, VERFAHREN ZU DEREN HERSTELLUNG UND IHRE VERWENDUNG**
MONOCLONAL ANTIBODIES AGAINST THE YPYDVPDYA EPITOPE, PROCESS FOR PRODUCING THE SAME AND THEIR USE
ANTICORPS MONOCLONAL CONTRE L'EPITOPE YPYVPDYA, SON PROCEDE DE PRODUCTION ET SON UTILISATION

(30) Priorität: 21.10.1996 DE 19643314
(43) Veröffentlichungstag der Anmeldung: 04.08.1999
(73) Patentinhaber: Roche Diagnostics GmbH, 68298 Mannheim (DE)
(72) Erfinder: EMRICH, Thomas, D-82393 Iffeldorf (DE); HINZPETER, Matthias, D-81375 München (DE); GROL, Michael, D-82340 Feldafing (DE)
(74) Vertreter: Jung, Michael, Dr.
(86) Internationale Anmeldenummer: EP9705783
(87) Internationale Veröffentlichungsnummer: WO98017691

(56) Entgegenhaltungen:
- WO-A-92/09300
- M. HINDS ET AL.: "Synthesis, conformational properties, and antibody recognition of peptides containing beta-turn mimetics based on alpha-alkylproline derivatives." JOURNAL OF MEDICINAL CHEMISTRY, Bd. 34, Nr. 6, Juni 1991, WASHINGTON, DC, USA, Seiten 1777-1789, XP002057282
- P. KOLODZIEJ ET AL.: "Epitope tagging and protein surveillance." METHODS IN ENZYMOLOGY, Bd. 194, 1991, NEW YORK, NY, USA, Seiten 508-519, XP002057283 in der Anmeldung erwähnt
- J. FIELD ET AL.: "Purification of a RAS-responsive adenylyl cyclase complex from Saccharomyces cerevisiae by use of an epitope addition method." MOLECULAR AND CELLULAR BIOLOGY, Bd. 8, Nr. 5, Mai 1988, WASHINGTON, DC, USA, Seiten 2159-2165, XP002057284 in der Anmeldung erwähnt
- Y. CHEN ET AL.: "Expression and localization of two low molecular weight GTP-binding proteins, Rab8 and Rab10, by epitope tag." PROCEEDINGS OF THE NATIONAL ACADEMY OF SCIENCES OF THE USA, Bd. 90, Nr. 14, 15.Juli 1993, WASHINGTON, DC, USA, Seiten 6508-6512, XP002057285 in der Anmeldung erwähnt

## Beschreibung

Die Erfindung betrifft monoklonale Antikörper gegen das Epitop YPYDVPDYA, das aus dem Hämagglutinin des humanen Influenzavirus abgeleitet ist und die zum Nachweis und zur Isolierung von nativem Hämagglutinin des humanen Influenzavirus, von modifiziertem Hämagglutinin oder von Hämagglutinin-Fusionsproteinen geeignet sind sowie eine Affinität> 10⁸ M⁻¹, insbesondere von 10⁹ bis 10¹⁰ M⁻¹ aufweisen.

Hämagglutinine sind Substanzen - meist Glykoproteine - mit der Fähigkeit, Erythrocyten zu agglutinieren. Sie kommen u.a. als Bestandteile von Viren, wie z.B. in Myxoviren oder Pockenviren vor. Von besonderer Bedeutung ist das Hämagglutinin (HA) des Influenzavirus, einem membranumhüllten Virus mit einem (-) RNA-Genom. Das Influenza-Hämagglutinin ist ein transmembranales Oberflächenantigen, das in Form von Spikes aus der sphärischen Lipidhülle herausragt, wie man auf elektronenmikroskopischen Aufnahmen erkennen kann. Die HA-Spikes sind Trimere, deren Monomere aus zwei Polypeptidketten bestehen, HA1(46.000-65.000 D) und HA2(21.000-30.000 D). Das Hämagglutinin in der Membran des Influenzavirus ermöglicht es dem Virus, in empfängliche Wirtszellen, z.B. des Respirationstraktes, einzudringen.

Bekanntermaßen sind Antikörper gegen Hämagglutinin effektive Hemmer der Virusinfektion. Die spezifische Affinität des Antikörpers gilt im allgemeinen jedoch nicht fiir das gesamte makromolekulare Protein, sondern nur für ein spezielles Epitop.

Um Proteine zu analysieren, ist heute die Technik des Epitop-Taggings, d.h. das Anfügen eines Epitops an ein Protein durch molekularbiologische Techniken, eine häufig angewandte Methode und prinzipiell unabhängig vom verwendeten Epitop. Dabei wird die Primärsequenz eines beliebigen Proteins mit Hilfe rekombinanter Techniken um einige wenige Aminosäuren erweitert. Ausschlaggebend ist lediglich das Vorhandensein eines spezifischen und hochaffinen Antikörpers mit bekannter Erkennungssequenz. Mit Hilfe von Antikörpern, die spezifisch gegen den erweiterten Proteinanteil gerichtet sind, bietet diese Methode die Möglichkeit, z.B. das Molekulargewicht eines Proteins, seine zelluläre Lokalisation, posttranslationale Modifizierungen oder Wechselwirkungen mit anderen Faktoren zu analysieren, ohne daß Protein-spezifische Antikörper vorhanden sein müssen.

Virale Epitope haben dabei gegenüber zellulären Epitopen den Vorteil, daß diese Proteinsequenzen in bakteriellen und eukaryontischen Proteinen in der Regel nicht vorkommen und damit keine Kreuzreaktionen in bakteriellen oder zellulären Systemen zu erwarten sind.

Ein häufig in der Literatur beschriebenes virales Epitop, das für derartige Analysen verwendet wird, ist aus dem Hämagglutinin des humanen Influenzavirus abgeleitet. Dieses Epitop weist die Aminosäuresequenz YPYDVPDYA (98-106) auf (Field, J. et al (1988), Mol. Cell. Biol. Vol. 8, Nr. 5, 2159-2165 und Wilson et al., Cell 37, 767 - 778, 1984). Monoklonale Antikörper (mAK) gegen dieses Epitop sind beschrieben und verfügbar, so z.B. der mAK 12CA5 (P.A. Kolodziej und Young, R.A., Meth. Enzymol. (1991), Vol. 194, 508-519; Chen, Y.-T. et al (1993), Proc. Natl. Acad. Sci., Vol. 90, 6508-6512)), die mAKs DB 19/1 und DB 19/25 (M.G. Hinds et al., J. Med. Chem. (1991), Vol. 34, 1777-1789), der mAK 19B10 (WO 92/09300) und der Anti-HA-BabCo.

Diese Antikörper besitzen jedoch den Nachteil, daß ihre Affinität nicht genügend hoch ist, was dazu führt, daß für eine sensitive Detektion entsprechender Fusionsproteine der Epitop-spezifische Antikörper in einer hohen Konzentration eingesetzt werden muß, wodurch unspezifische Wechselwirkungen hervorgerufen werden können, die z.B. als Kreuzreaktionen im Western-Blot deutlich werden (vgl. Chen et al., S. 6510).

Bedingt durch die unzureichende Affinität ist auch eine geringere Sensitivität der bekannten Anti-HA-mAK zu verzeichnen.

Der Erfindung lag deshalb die Aufgabe zugrunde, monoklonale Antikörper gegen das virale Epitop YPYDVPDYA bereitzustellen, die eine höhere Affinität aufweisen und die somit zu hochsensitiven Hämagglutinin-Nachweisen oder HA-Fusionsprotein-Nachweisen geeignet sind und reproduzierbare Ergebnisse liefern.

Erfindungsgemäß wurden monoklonale Antikörper bereitgestellt, die das Epitop mit der Aminosäuresequenz YPYDVPDYA (98-106) des Hämagglutinis des humanen Influenzavirus sowie entsprechende Fragmente hiervon erkennen und eine Affinität von 10⁹ - 10¹⁰ M⁻¹ (ermittelt mittels Plasmonresonanz) aufweisen und mittels des HA-Peptids Acetyl-YPYDVPDYAGSGSK (ε-Biotinoyl)-Amid oder Biotinoyl-ε-Aca-SGSGYPYDVPDYA-Amid erhalten wurden. Unter Epitopfragmenten werden dabei insbesondere solche Aminosäuresequenzen verstanden, die mindestens 70% der Sequenz YPYDVPDYA entsprechen bzw. mindestens um ein bis zwei terminale Aminosäuren verkürzt sind.

Zur Herstellung der monoklonalen Antikörper wurden Kleinsäuger, vorzugsweise Ratten, wie z.B. Lou/C-Ratten oder Mäuse, wie z.B. BalbC-Mäuse, oder Kaninchen, mit einem nach Standardmethoden synthetisierten HA-Peptid immunisiert. Es wird als Antigen ein ungekoppeltes HA-Peptid oder ein HA-Peptid, das gegebenenfalls über ein Carrier-Protein N- oder C- terminal gekoppelt ist oder ein HA-Fusionsprotein eingesetzt. Vorzugsweise wurden als Carrier-Proteine Keyhole Limpet Hämocyanin (KLH) oder Bovines Serumalbumin (BSA) verwendet. Anschließend wurden B-Lymphozyten aus der Milz der Tiere isoliert und durch Zellfusion mit geeigneten Myelomzellen oder nach anderen an sich bekannten Verfahren, wie z.B. mittels Onkogene (Jonak, Z.L. et al, (1988) Adv. Drug Rev. 2:207-228) oder in einem elektrischen Feld (Zimmermann, U. (1982) Biochim. Biophys. Acta 694:227-277) immortalisiert. Erfindungsgemäß bevorzugt wurde die Zellfusion mit Milzzellen von Lou/C-Ratten und Myelomzellen der Linie Maus P3x63-Ag8,653 (Kearney, J.F. et al (1979), J. Immunol. 123, 1548-1550) durchgeführt.

Dabei werden die Lymphozyten und die Myelomzellen nach den bekannten Verfahren, insbesondere Polyethylenglykolfusion (PEG), Virus-Fusion oder Elektrofusion fusioniert und entstehende Hybridzellen (Zellklone) nach ebenfalls bekannten Verfahren, wie z.B. durch Einsatz von Selektionsmedien, selektiert.

So wurden beispielsweise positive Klone zunächst mit HA-Peptiden und dann mit HA-Fusionsproteinen getestet. In einem ersten Screen wurde ein biotinoyliertes HA-Peptid, z.B. Bio-C-HA (Acetyl-**YPYDVPDYA**GSGSK(ε-Bionoyl)-Amid) oder Bio-N-HA (Biotinoyl-ε-Aca-SGSG**YPYDVPDYA**-Amid), und in einem zweiten Screen eine HA-getaggte Glutathion-S-Transferase (GST) verwendet. Wiederum positive Klone wurden anschließend mit Hilfe der Plasmonresonanz in einem BiaCore-System auf ihre Affinität untersucht und selektioniert.

Die Hybridzellen wurden nach bekannten Verfahren kloniert, kultiviert und vermehrt und gegebenenfalls in flüssigem Stickstoff aufbewahrt.

Als aktivste Zellklone mit stabiler Antikörperproduktion wurden die Zellinien R 3F10, R 3A12 und R 6D12 etabliert. Das Hybridom R 3A12 wurde in der Deutschen Stammsammlung für Mikroorganismen und Zellkulturen (DSMZ), Mascheroder Weg 1b, 38124 Braunschweig unter der Nummer DSM ACC2286 am 08.10.1996 hinterlegt.

Zur Antikörpergewinnung wurden die Hybridzellen in der Zellkultur oder gegebenenfalls in vivo durch Tranplantation als Ascitestumore weitervermehrt. Die mAK wurden aus den Zellkulturüberständen oder gegebenenfalls aus der Ascitesflüssigkeit der tumortragenden Versuchstiere isoliert.

Erfindungsgemäß werden die von den Hybridzellen in hoher Konzentration produzierten mAK, die sich durch ausgezeichnete Spezifizität und Bindungsstärke für das Epitop YPYDVPDYA des Hämagglutinins des humanen Influenzavirus bzw. für entsprechende Epitop-Fragmente auszeichnen, erhalten. Sie ermöglichen den hochsensitiven Nachweis und die Isolierung von Hämagglutinin sowie von Proteinen, an die das HA-Epitop YPYDVPDYA angefügt wurde.

Die Affinität der erfindungsgemäßen mAK liegt bei > 10⁸ M⁻¹. So erwies sich die Affinität des mAK 3F10 mit 10¹⁰ M⁻¹ ungefähr um das 30-fache höher als die der bekannten Antikörper 12CA5 (10⁸M⁻¹) und BabCo (10⁷ M⁻¹).
Die Affinität der erfindungsgemäßen mAK 3A12 und 6D12 beträgt 10⁹M⁻¹ und liegt damit ebenfalls über der der bekannten Antikörper. Die erfindungsgemäßen mAK sind in wesentlich geringeren Konzentrationen einsetzbar und Kreuzreaktionen sind nahezu auszuschließen. Sie ermöglichen eine verbesserte Sensitivität der Detektion. Es zeigte sich, daß sie sowohl natives HA des Influenzavirus, modifiziertes HA als auch HA-Fusiohsproteine erkennen. Sie sind somit in an sich bekannten Nachweisreaktionen, wie z.B. dem Festphasen-Zwei-Seiten-Bindungstest zur Bestimmung von Proteinen sehr gut einsetzbar.

### Legenden zu den Abbildungen

### Abbildung 1:

Affinitäten der erfindungsgemäßen mAK im Vergleich zu den mAK 12CA5 und Anti-HA BabCo.

### Abbildung 2:

Immunoblot-Analyse eines HA-modifizierten Glutathion-S-transferase-Proteins mit erfindungsgemäßen mAK (Klon 3F10) und anti-HA nach dem Stand der Technik (Klon 12CA5); a) Detektion mit anti-Ratte-Peroxidase, b) anti-Ratte-Biotin/Streptavidin-Peroxidase.

### Abbildung 3:

Immunoblot-Analyse eines HA-modifizierten Glutathion-S-transferase-Proteins mit erfindungsgemäßem mAK (Klon 3F10) und anti-HA nach dem Stand der Technik (Klon 12CA5).

### Abbildung 4:

Immunoblot-Analyse eines HA-modifizierten Glutathion-S-transferase-Proteins mit Enzym (Peroxidase-) Konjugaten eines erfindungsgemäßen Antikörpers (Klon 3F10) und eines Antikörpers entsprechend dem Stand der Technik (Klon 12CA5).

### Abbildung 5:

Immunpräzipitation eines HA-modifizierten Green-Fluorescent (GFP-HA)-Proteins mit erfindungsgemäßem mAK (Klon 3F10) und anti-HA nach dem Stand der Technik (Klon 12CA5).

Anschließend wird die Erfindung an folgenden Ausführungsbeispielen näher erläutert.

### Beispiel 1:

### Herstellung der Klone R 3F10, R 3A12 und R 6D12

### HA-Peptid-Herstellung

Folgende Peptide wurden synthetisiert:
Bio-C-HA (Acetyl-**YPYDVPDYA**GSGSK(ε-Biotinoyl)-Amid)
Bio-N-HA (Biotinoyl-ε-Aca-SGSG**YPYDVPDYA**-Amid)
KLH-MPS-CUZU-HA-C
KLH-MPS-CUSU-HA-N

### Immunisierung von Kleinsäugern

Lou/c-Ratten wurden intraperitoneal mit KLH-gekoppeltem HA-Peptid nach folgendem Schema immunisiert:

Zu einer Grundimmunisierung der Tiere wurden 50 µg KLH-gekoppeltes HA-Peptid in komplettem Freund schen Adjuvans injiziert.

Weitere Immunisierungen wurden mit 50 µg KLH-gekoppeltem HA-Peptid in inkomplettem Freund'schen Adjuvans durchgeführt.

### Fusion

Die sich anschließende Fusion der Milzzellen von den Lou/C-Ratten erfolgte mit Maus P3x63-Ag8,653 in Gegenwart von PEG nach Kremmer et al (1990) Hybridoma 9, 309-317.

### Durchführung der Selektion der Klone

Screening-Schema:

### 1. Screen

- Eine SA-beschichtete Mikrotiterplatte (MTP) wurde mit 1 µg/ml Bio-C-HA bzw. Bio-N-HA beschichtet,
- Hybridomaüberstände wurden unverdünnt verwendet und in die beschichtete MTP gegeben,
- die Detektion der gebundenen Antikörper erfolgte mit Hilfe von Anti-Ratte-POD-Konjugat/TMB-Substrat.

### 2. Screen

- Eine Maxisorb-MTP wurde mit HA-getaggter GST (1 µl/ml in Carbonat-Puffer) beschichtet,
- die beim 1. Screen ausgewählten Hybridomaüberstände wurden wiederum unverdunnt verwendet und in die beschichtete MTP gegeben,
- die Detektion der gebundenen Antikörper erfolgte mit Hilfe von Anti-Ratte-POD-Konjugat/TMB-Substrat.

### 3./4. Screen

- Es wurden BiaCore-Messungen mit analoger Beschichtung durchgeführt.

### Ergebnis

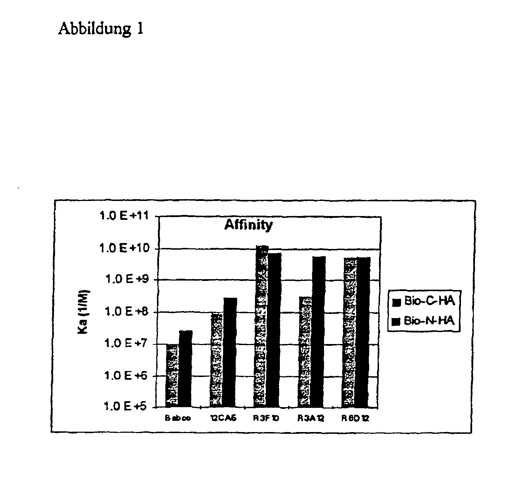

Mit Hilfe der Plasmonresonanz in dem BiaCore-System wurden 5 Klone mit der höchsten Affinität und der längsten Halbwertszeit der Dissoziation selektioniert. Sie wurden mit R 3F10, R 3A12, R 6D12, R 4H10 und M5B9 bezeichnet. Die Affinität war nur geringfügig unterschiedlich in Abhängigkeit der biotinoylierten Position der Peptide (C-oder N-Terminus - vgl. Abb. 1).

Die Klone R 3F10, R 3A12 und R 6D12 wurden als Zellinien etabliert. Sie zeigen ein gutes Wachstum und eine stabile Antikörperproduktion, wobei die produzierten Antikörper eine um den Faktor 10 bis 100 höhere Affinität als die monoklonalen Antikörper des Standes der Technik 12CA5 und Anti-HA BabCo aufweisen.

Abb. 1 zeigt die Affinitäten der erfindungsgemäßen mAK im Vergleich zu den mAk 12CA5 und Anti-HA BabCo.

### Beispiel 2

### Bestimmung der Affinitätkonstanten sowie der Geschwindigkeitskonstanten der Assoziation und Dissoziation der produzierten Antikörper

Die Bestimmung der Affinitätskonstanten sowie der Geschwindigkeitskonstanten der Assoziation und Dissoziation der produzierten Antikörper erfolgte mit BIAcore® der Firma Pharmacia Biosensor (BIA steht für Biospezifische Interaktions Analyse). Das Messprinzip beruht auf der "Surface Plasmon Resonance". Die Messung wird auf einem Biosensor, dem sog. Sensorchip durchgeführt. An den mit Streptavidin beschichteten Sensorchip wird das biotinylierte Peptid durch eine nichtkovalente, hochaffine Bindung gekoppelt. Über diesen Sensorchip wird eine Lösung des zu untersuchenden Antikörpers geleitet, wobei der Antikörper durch nichtkovalente Wechselwirkungskräfte an das immobilisierte Peptid gebunden wird.

Durch die Bindung der einzelnen Komponenten erhöht sich die Massendichte auf der Oberfläche des Sensorchips, die vom Gerät in ein proportionales Messignal umgewandelt wird. Aus der zeitlichen Änderung des Signals, dem Sensorgramm, lassen sich die Geschwindigkeitskonstanten der Assoziation und Dissoziation und daraus die Affinitätskonstante berechnen.

Die Antikörper-Peptid-Komplexe lassen sich ohne Beeinträchtigung der an die Oberfläche gebundenen Peptide mit einfachen Mitteln wieder ablösen, so daß an demselben Sensorchip weitere Bindungsexperimente unter identischen Randbedingungen durchgeführt werden können.

Zur Kopplung der biotinylierten Peptide an den Sensorchip (SA, Pharmacia Biosensor) wird eine Lösung mit einer Konzentration von 50 nMol/l in HBS (10 mMol/l HEPES, 150 mMol/l NaCl 3,4 mMol/l EDTA 0,05 % P20 pH 7,4) über den Sensorchip mit einer Flußrate von 5 ml/min geleitet.
Danach werden die Antikörper in HBS zugegeben und die Bindung an die Peptide bei einer Flußrate von 10 µl/min verfolgt. Aus den Sensorgrammen werden die Geschwindigkeitskonstanten der Assoziation und der Dissoziation der Bindung der Antikörper an die Peptide mit Hilfe einer Software des Herstellers (BIAevaluation 2.1, Pharmacia Biosensor) berechnet. Die Affinitätskonstante berechnet sich nach Ka = kon/koff. Die so ermittelten Werte der erfindungsgemäßen Antikörper an Bio-C-HA und BioN-HA als Antigene sind in Tabelle 1 zusammengefaßt.

**Tabelle 1**

| anti-HA-mAb | Antigen | kon l/mol*s | koff 1/s | Ka l/mol |
|---|---|---|---|---|
| Babco | Bio-C-HA | 9.5 E +3 | 1.0 E -3 | 9.3 E +6 |
| 12CA5 | Bio-C-HA | 2.3 E +4 | 2.6 E -4 | 9.2 E +7 |
| R3F10 | Bio-C-HA | 5.9 E +5 | 4.8 E -5 | 1.2 E +10 |
| R3A12 | Bio-C-HA | 1.7 E +5 | 5.4 E -4 | 3.1 E *8 |
| R6D12 | Bio-C-HA | 4.0 E +5 | 7.6 E -5 | 5.2 E +8 |
| Babco | Bio-N-HA | 1.2 E +4 | 5.0 E -4 | 2.3 E +7 |
| 12CA5 | Blo-N-HA | 5.3 E +4 | 2.1 E -4 | 2.6 E +8 |
| R3F10 | Bio-N-HA | 6.2 E +5 | 9.1 E -5 | 6.8 E +9 |
| R3A12 | Bio-N-HA | 7.7 E +5 | 1.4 E -4 | 5.7 E +9 |
| R6D12 | Bio-N-HA | 5.9 E +5 | 1.2 E -4 | 5.1 E +9 |

### Beispiel 3

### Vergleichende Immunoblot-Analyse eines HA-modifizierten Proteins unter Verwendung eines erfindungsgemäßen Antikörpers und eines aus dem Stand der Technik bekannten Antikörpers

### 3.1. Variation des Antigens

Mit dem HA-Epitop modifizierte Glutathion-S-transferase (GST-HA) wurde seriell auf die angegebenen Mengen verdünnt, mittels SDS-Polyacrylamidgelelektrophorese separiert und nach Transfer auf eine Nylon-Membran mit Hilfe der angegebenen Antikörper in den angegebenen Konzentrationen der Primärantikörper umgesetzt (1µg/µl Klon 12CA5; 0.1 µg/ml Klon 3F10; Western-Blot-Analyse). Gebundene anti-HA-Antikörper wurden anschließend unter Verwendung von anti-Maus-Peroxidase (bei Klon 12CA5), anti-Ratte-Peroxidase (bei Kleon 3F10, a)) bzw. anti-Ratte-Biotin/Streptavidin-Peroxidase (bei Klon 3F10, b)) und Chemilumineszenz-Detektion nachgewiesen.

### SDS-Polyacrylamidgelelektrophorese

Es wurden 8 x 7 cm große Gele mit 0.75 mm Dicke verwendet (BIO-RAD, Mini-Protean II ™). Für ein 15%-iges Gel wurden 6 ml Trenngellösung (1x) zwischen die Platten gegossen. Die Trenngeloberkante wurde vorsichtig mit 1 ml Wasser überschichtet. Nach 30 min. Polymerisation wurde das Wasser entfernt und 2 ml Sammelgellösung (1x) eingefüllt. Die Proben wurden mit 1 Volumen zweifach konzentriertem Laemmli-Puffer vermischt, 10 min. bei 60°C inkubiert und in die gespülten Geltaschen pipettiert. Die Elektrophorese erfolgte in Laufpuffer bei einer Stromstärke von ca. 15 mA.

### Lösungen:

| **4 x Trenngellösung:** | | |
|---|---|---|
| 1,5 | M | Tris/HCl, pH 8,8 |
| 0,4 | % | SDS |
| | | |

| **4 x Sammelgellösung:** | | |
|---|---|---|
| 0,5 | M | Tris/HCL, pH 6,8 |
| 0,4 | % | SDS |
| | | |

| **2 x Laemmli-Puffer:** | | |
|---|---|---|
| 1,52 | g | Tris pro 100 ml |
| 10 | ml | Glyzerin pH 6,8 mit 1 N HCI eingestellt |
| 2,0 | g | SDS |
| 2,0 | ml | 2-Mercaptoethanol |
| 1 | mg | Bromphenolblau |
| | | |

| **5 x Laufpuffer:** | | |
|---|---|---|
| 15,1 | g | Tris pro 11 |
| 72,0 | g | Glycin |
| 5,0 | g | SDS |

### Transfer auf Nylon-Membran

Nach der elektrophoretischen Auftrennung der Proteinproben wurde ein der Gelgröße entsprechender, mit H₂O befeuchteter Nylon-Filter auf das Gel aufgelegt. Zum besseren Kontakt wurden beidseitig je zwei zugeschnittene Whatman®-3 MM-Papiere aufgelegt und der Aufbau wurde in die Vorrichtung einer BIO-RAD Elektroblot-Apparatur eingespannt. Nach Füllen der Kammer mit Transfer-Puffer wurden die Proteine unter Eiskühlung und Rühren 45 - 60 min durch Anlegen einer Spannung von 70 V (Stromstärke, I=250-350 mA) auf die Membran transferiert.

### Lösung:

| | | | |
|---|---|---|---|
| **Transfer-Puffer** | 25 | mM | Tris/HCI, pH 8,3 |
| | 192 | mM | Glyzin |

### Detektion

Nach vorsichtigem Entfernen der Membran wurde der Filter zur Absättigung noch freier Bindungsstellen 60 min bei RT in PBS-Lösung unter Zusatz von 1% Blocking-Reagenz (PBS/PR; Boehringer Mannheim) inkubiert. Es wurde zweimal 10 min in PBS-Puffer unter Zusatz von 0.1% Tween-20 (PBST) gewaschen und 60 min mit dem entsprechenden anti-HA-Antikörper in den angegebenen Konzentrationen bei RT umgesetzt. Nach dreimaligem Waschen in PBST-Puffer (je 5 min, RT) wurde der Filter weitere 60 min mit einem Anti-Spezies-IgG-Peroxidase-Konjugat (anti-Ratte-Peroxidase, 20 mU/ml PBS/BR; anti-Maus-Peroxidase, 40 mU/ml PBS/BR) bei RT inkubiert. Es wurde wiederum, wie oben beschrieben, gewaschen, und die Filtermembran nach Entfernung des Puffers mit einem saugfähigen Tuch in einer 1:100-Mischung von Detektionsreagenz A+B (Boehringer Mannheim) 1 min inkubiert, anheftendes Reagenz entfernt. Mit dem mit Haushaltsfolie abgedeckten Filter wurde anschließend ein Röntgenfilm für 1 - 10 min exponiert.

### Ergebnis

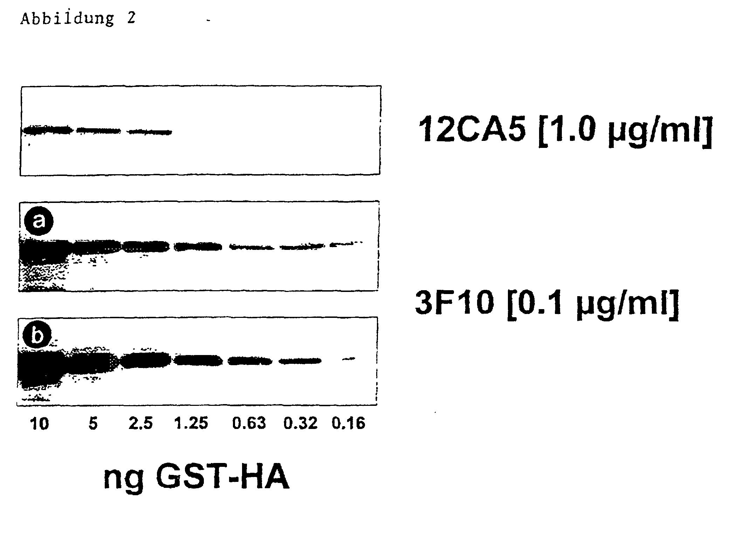

Mit einer um das 10-fache geringeren Antikörperkonzentration kann mit erfindungsgemäßen Antikörper, beispielsweise Klon 3F10 im Vergleich zu bekannten Antikörper wie Klon 12CA5 ein um den Faktor 30 geringere Menge an GST-HA detektiert werden (Abb. 2).

### 3.2 Variation der Konzentration des Primärantikörpers

Pro Spur wurden 4ng mit dem HA-Epitop modifizierte Glutathion-S-transferase (GST-HA) mittels SDS-Polyacrylamidgelelektrophorese separiert und nach Transfer auf eine Nylon-Membran mit dem angegebenen Antikörper in den angegebenen Konzentrationen der Primärantikörper umgesetzt (Klone 12CA5 und 3F10; 2.0 - 0.008 ng/ml). Gebundene anti-HA-Antikörper wurden anschließend unter Verwendung von anti-Mäus-Peroxidase (bei Klon 12CA5) bzw. anti-Ratte-Peroxidase (bei Klon 3F10) und Cheinilumineszenz-Detektion nachgewiesen.

SDS-Polyacrylamidgelelektrophorese, Transfer auf eine Nylon-Membran und anschließende Detektion erfolgten wie in Beispiel 3.1 beschrieben.

### Ergebnis:

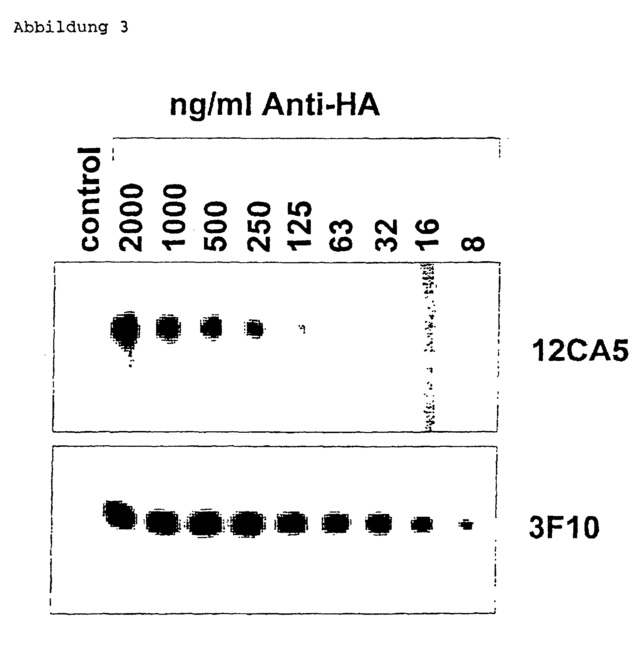

Im beschriebenen Experiment wird Klon 12CA5 (Stand der Technik) bei einer Antikörperkonzentration von ≤ 2000 ng/ml zum limitierenden Faktor, während mit dem erfindungsgemäßen Klon 3F10 noch bei einer Antikörperkonzentration von ca. 125 ng/ml Signalsättigung beobachtet wird (Abb. 3).

Vergleichbare Signale werden im Falle von Klon 3F10 mit ca. 20-fach geringeren Antikörperkonzentrationen erreicht.

### Beispiel 4

### Vergleichende Analyse Immunoblot-Analyse eines HA-modifizierten Proteins mit Antikörper-Konjugate

Mit dem HA-Epitop modifizierte Glutathion-S-transferase (GST-HA) wurde seriell auf die angegebenen Mengen verdünnt, mittels SDS-Polyacrylamidgelelektrophorese separiert und nach Transfer auf eine Nylon-Membran mit Hilfe der angegebenen Antikörper-Peroxidase-Konjugaten in den angegebenen Konzentrationen umgesetzt (100 ng/ml (Klon 12CA5); 20 mU/ml (Klon 3F10)). Gebundene anti-HA-Antikörper wurden anschließend-unter Verwendung von Chemilumineszenz-Detektion nachgewiesen.

SDS-Polyacrylamidgelelektrophorese, Transfer auf eine Nylon-Membran und anschließende Detektion erfolgte wie in Beispiel 3.1 beschrieben.

### Ergebnis:

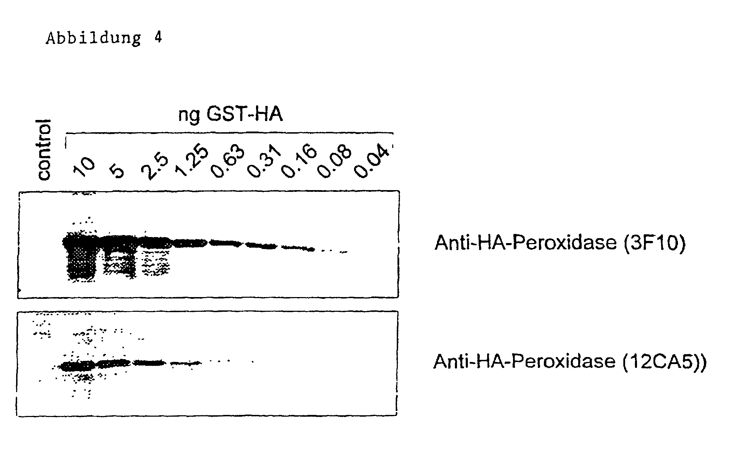

Die Detektionsgrenze in diesem System liegt für das Peroxidase-Konjugat des Klons 12CA5 (= Stand der Technik) bei ca. 1 ng GST-HA, während unter den gleichen Bedingungen mit dem entsprechenden Derivat des Klons 3F10 noch 40 pg GST-HA nachgewiesen werden können (Abb. 4).

### Beispiel 5

### Vergleichende Immunpräzipitation eines HA-modifizierten Proteins

Mit dem HA-Epitop modifiziertes Green-Fluorescent-Protein (GFP-HA) wurde mit den angegebenen Mengen [in µg] an anti-HA-Antikörper (Klon 12CA5 bzw. Klon 3F10) umgesetzt und nach Zugabe von Protein-G-Agarose immunpräzipitiert. Die erhaltenen Präzipitate wurden solubilisiert, mittels SDS-Polyacrylamidgelektrophorese separiert und nach Transfer auf eine Nylon-Membran mit einem anti-HA-Peroxidase-konjugat (Klon 12CA5) und Chemilumineszenz-Detektion nachgewiesen.

### Immunpräzipitation

50 ng mit dem HA-Epitop modifiziertes Green-Fluorescent-Protein (GFP-HA) wurden in 200 µl Waschpuffer verdünnt, mit den angegebenen Mengen an anti-HA-Antikörper versetzt und 1 h bei 4° C im Überkopfschüttler inkubiert. Dem Ansatz wurden daraufhin 25µl einer 50%-igen Protein-G-Sepharose-Suspension zugefügt und es wurde erneut 1 h wie zuvor inkubiert. Über Protein-G an die Gelmatrix gebundene Proteine wurden dreimal mit je 1 ml Waschpuffer gewaschen, wobei bei jedem Waschschritt der Ansatz 2 min wie oben beschrieben bei 4° C inkubiert wurde. Der Ansatz wurde dann 2 min in einer Tischzentrifuge zentrifugiert (15 000 Upm, RT) und das Pellet in 10 ml 1xLaemmli-Auftragspuffer aufgenommen. Die immunpräzipierten Proteine wurden anschließend durch denaturierende Gelelektrophorese (SDS-Polyacrylamidgel) separiert.

SDS-Polyacrylamidgelelektrophorese, Transfer auf eine Nylon-Membran und anschließende Detektion erfolgte wie in Beispiel 3. 1 beschrieben.

### Ergebnis:

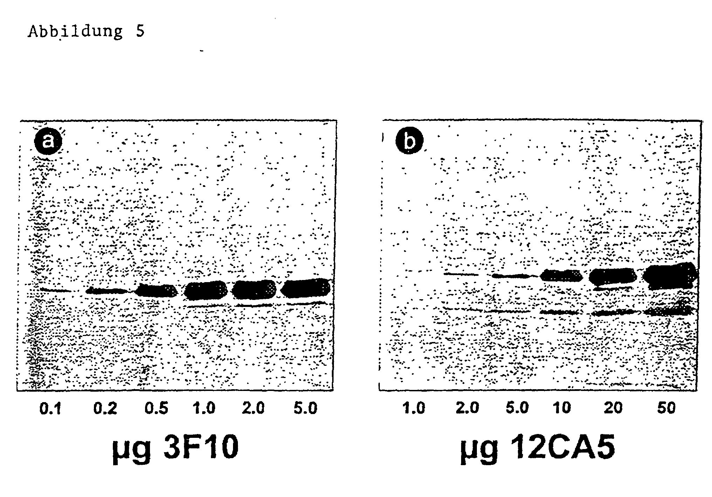

Im beschriebenen Experiment ist der Klon 3F10 im Vergleich zum Klon 12CA5 (Stand der Technik) in der Lage, bei Verwendung einer um den Faktor ≥ 20 geringeren Antikörpermenge das eingesetzte Antigen zu präzipitieren (Abb. 5).

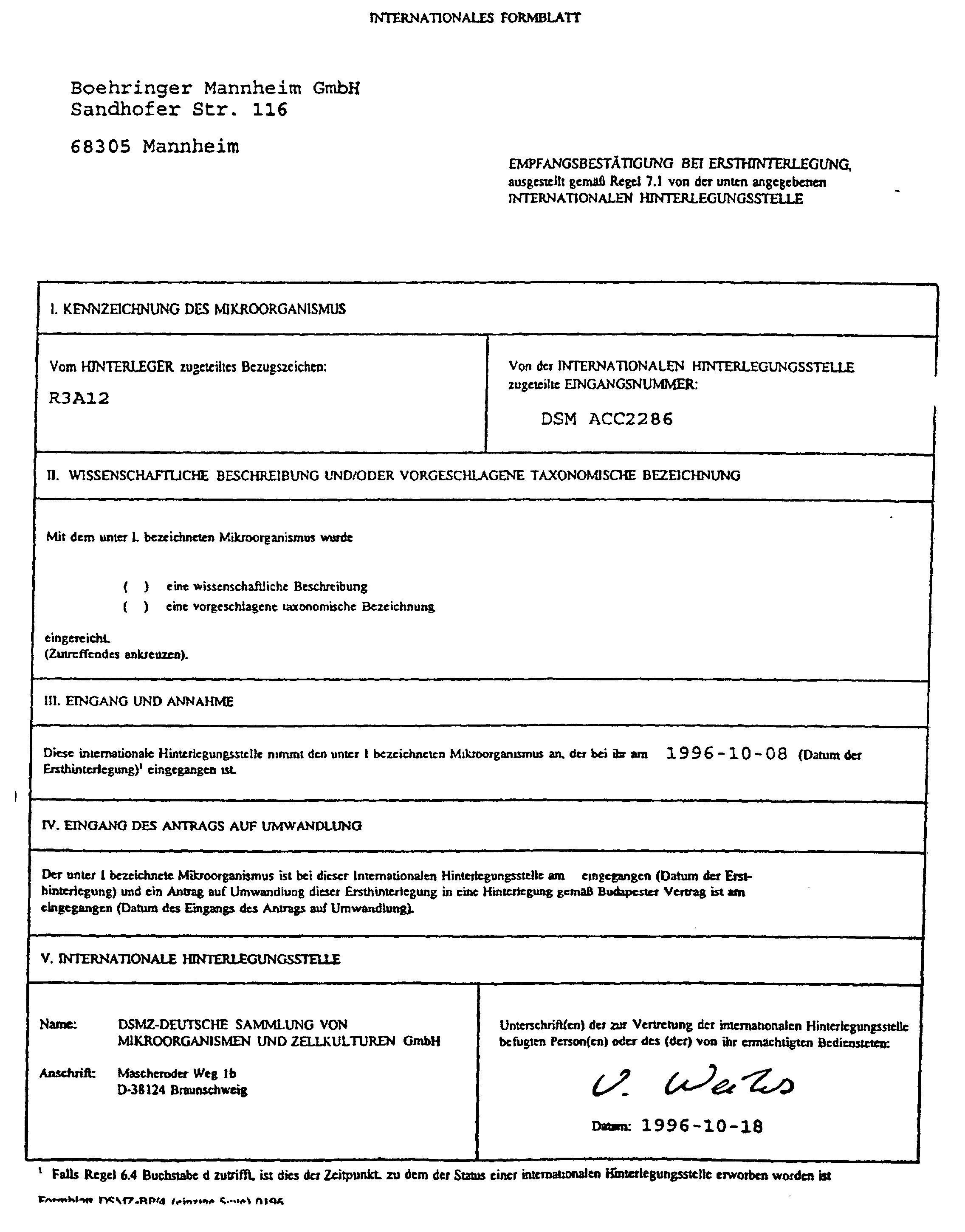
INTERNATIONALES FORMBLATT

Boehringer Mannheim GmbH
Sandhofer Str. 116

68305 Mannheim

EMPFANGSBESTÄTIGUNG BEI ERSTHINTERLEGUNG,
ausgestellt gemäß Regel 7.1 von der unten angegebenen
INTERNATIONALEN HINTERLEGUNGSSTELLE

I. KENNZEICHNUNG DES MIKROORGANISMUS

Vom HINTERLEGER zugeteiltes Bezugszeichen: | Von der INTERNATIONALEN HINTERLEGUNGSSTELLE zugeteilte EINGANGSNUMMER:
R3A12 | DSM ACC2286

II. WISSENSCHAFTLICHE BESCHREIBUNG UND/ODER VORGESCHLAGENE TAXONOMISCHE BEZEICHNUNG

Mit dem unter I. bezeichneten Mikroorganismus wurde

( ) eine wissenschaftliche Beschreibung
( ) eine vorgeschlagene taxonomische Bezeichnung

eingereicht.
(Zutreffendes ankreuzen).

III. EINGANG UND ANNAHME

Diese internationale Hinterlegungsstelle nimmt den unter I bezeichneten Mikroorganismus an, der bei ihr am 1996-10-08 (Datum der
Ersthinterlegung)¹ eingegangen ist.

IV. EINGANG DES ANTRAGS AUF UMWANDLUNG

Der unter I bezeichnete Mikroorganismus ist bei dieser Internationalen Hinterlegungsstelle am eingegangen (Datum der Erst-
hinterlegung) und ein Antrag auf Umwandlung dieser Ersthinterlegung in eine Hinterlegung gemäß Budapester Vertrag ist am
eingegangen (Datum des Eingangs des Antrags auf Umwandlung).

V. INTERNATIONALE HINTERLEGUNGSSTELLE

Name: DSMZ-DEUTSCHE SAMMLUNG VON
MIKROORGANISMEN UND ZELLKULTUREN GmbH

Anschrift: Mascheroder Weg 1b
D-38124 Braunschweig
| Unterschrift(en) der zur Vertretung der internationalen Hinterlegungsstelle befugten Person(en) oder des (der) von ihr ermächtigten Bediensteten:


Datum: 1996-10-18

¹ Falls Regel 6.4 Buchstabe d zutrifft, ist dies der Zeitpunkt, zu dem der Status einer internationalen Hinterlegungsstelle erworben worden ist

Formblatt DSMZ-BP/4 (einzige Seite) 0196

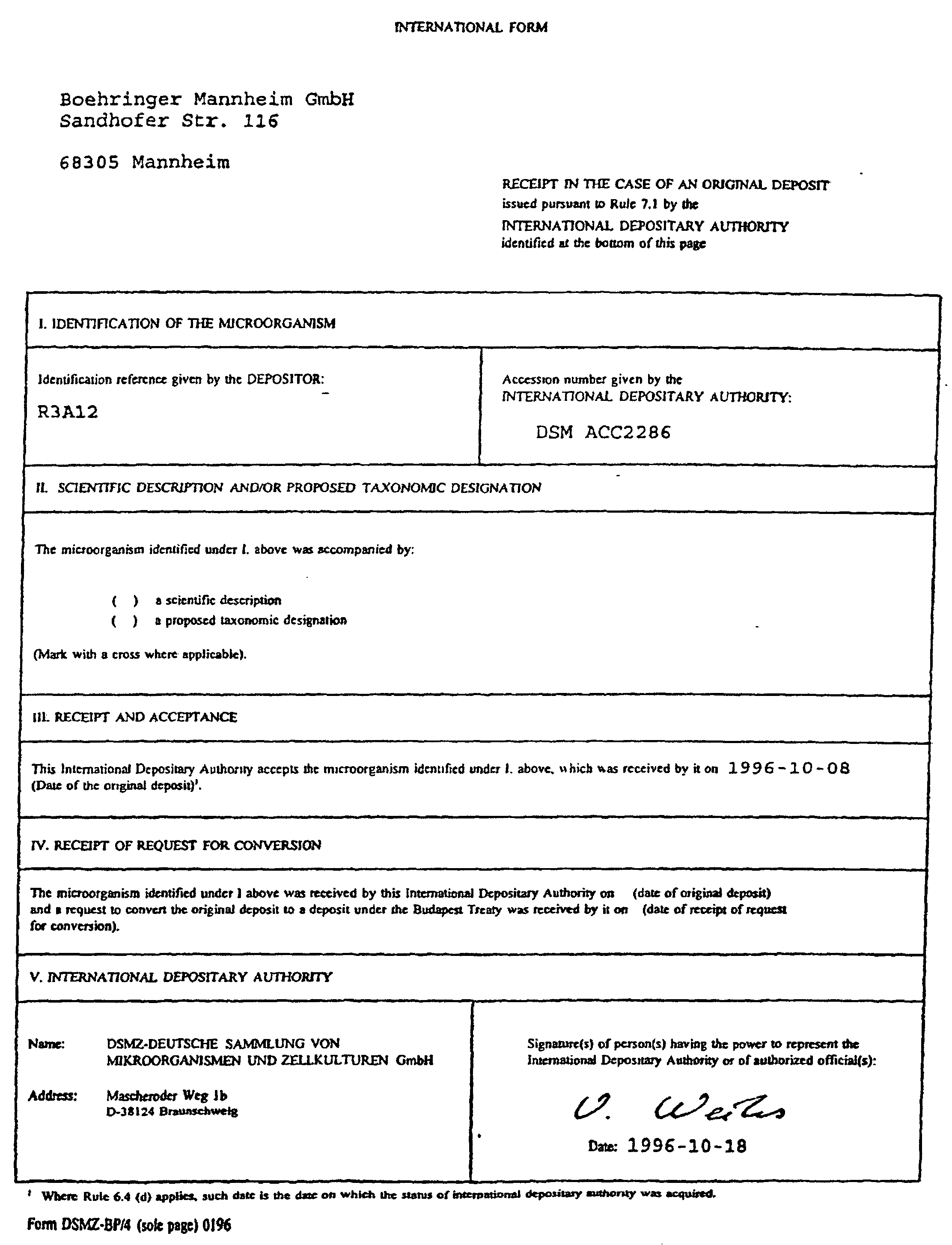
INTERNATIONAL FORM

Boehringer Mannheim GmbH
Sandhofer Str. 116

68305 Mannheim

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the DEPOSITOR: | Accession number given by the INTERNATIONAL DEPOSITARY AUTHORITY:
R3A12 | DSM ACC2286

II. SCIENTIFIC DESCRIPTION AND/OR PROPOSED TAXONOMIC DESIGNATION

The microorganism identified under I. above was accompanied by:

( ) a scientific description
( ) a proposed taxonomic designation

(Mark with a cross where applicable).

III. RECEIPT AND ACCEPTANCE

This International Depositary Authority accepts the microorganism identified under I. above, which was received by it on 1996-10-08
(Date of the original deposit)¹.

IV. RECEIPT OF REQUEST FOR CONVERSION

The microorganism identified under I above was received by this International Depositary Authority on (date of original deposit)
and a request to convert the original deposit to a deposit under the Budapest Treaty was received by it on (date of receipt of request
for conversion).

V. INTERNATIONAL DEPOSITARY AUTHORITY

Name: DSMZ-DEUTSCHE SAMMLUNG VON
MIKROORGANISMEN UND ZELLKULTUREN GmbH

Address: Mascheroder Weg 1b
D-38124 Braunschweig
| Signature(s) of person(s) having the power to represent the
International Depositary Authority or of authorized official(s):


Date: 1996-10-18

¹ Where Rule 6.4 (d) applies, such date is the date on which the status of international depositary authority was acquired.

Form DSMZ-BP/4 (sole page) 0196

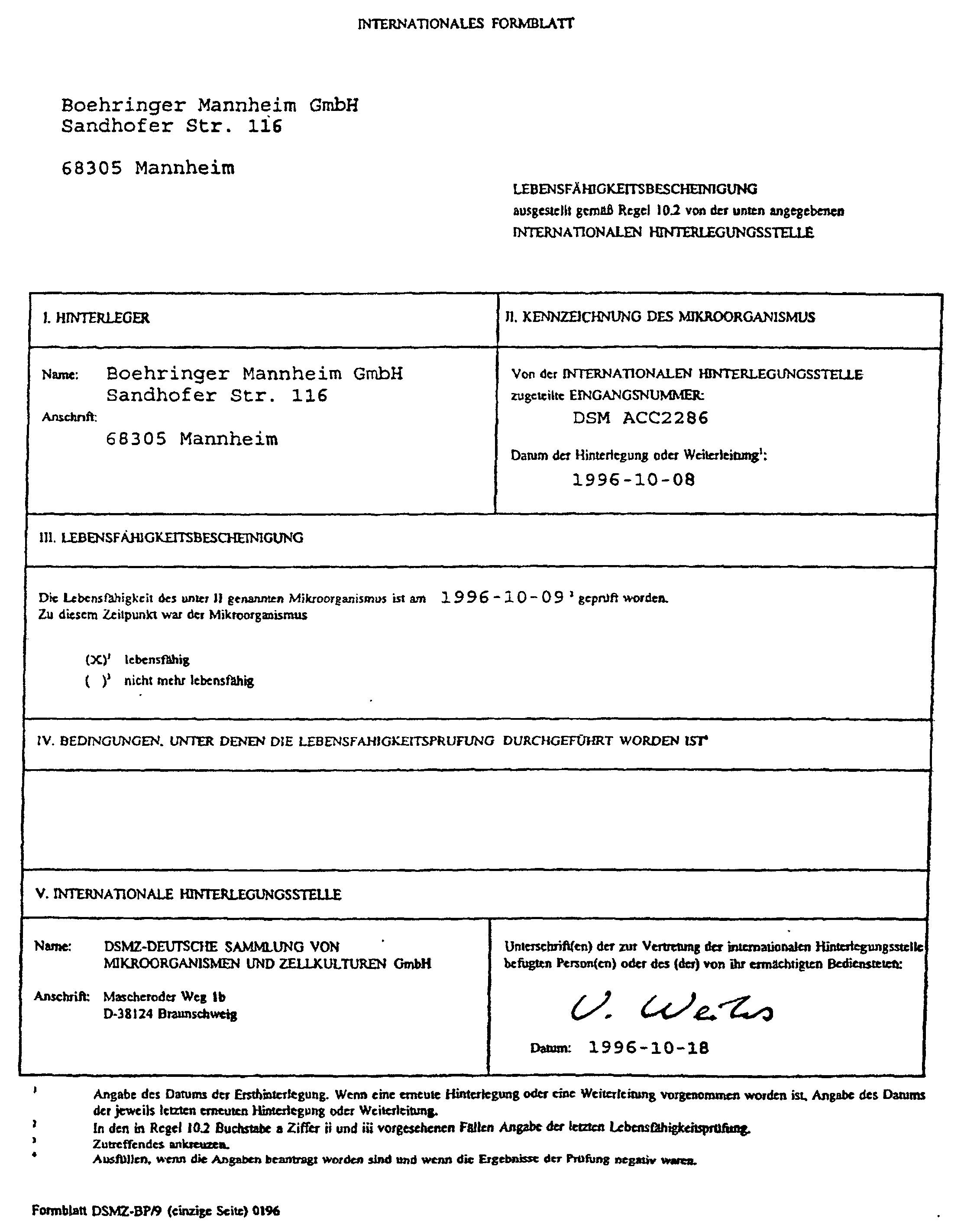
INTERNATIONALES FORMBLATT

Boehringer Mannheim GmbH
Sandhofer Str. 116

68305 Mannheim

LEBENSFÄHIGKEITSBESCHEINIGUNG
ausgestellt gemäß Regel 10.2 von der unten angegebenen
INTERNATIONALEN HINTERLEGUNGSSTELLE

I. HINTERLEGER | II. KENNZEICHNUNG DES MIKROORGANISMUS
Name: Boehringer Mannheim GmbH
Sandhofer Str. 116
Anschrift:
68305 Mannheim | Von der INTERNATIONALEN HINTERLEGUNGSSTELLE
zugeteilte EINGANGSNUMMER:
DSM ACC2286
Datum der Hinterlegung oder Weiterleitung¹:
1996-10-08

III. LEBENSFÄHIGKEITSBESCHEINIGUNG

Die Lebensfähigkeit des unter II genannten Mikroorganismus ist am 1996-10-09 ² geprüft worden.
Zu diesem Zeitpunkt war der Mikroorganismus

(X)³ lebensfähig
( )³ nicht mehr lebensfähig

IV. BEDINGUNGEN, UNTER DENEN DIE LEBENSFÄHIGKEITSPRÜFUNG DURCHGEFÜHRT WORDEN IST⁴

V. INTERNATIONALE HINTERLEGUNGSSTELLE

Name: DSMZ-DEUTSCHE SAMMLUNG VON
MIKROORGANISMEN UND ZELLKULTUREN GmbH
Anschrift: Mascheroder Weg 1b
D-38124 Braunschweig | Unterschrift(en) der zur Vertretung der internationalen Hinterlegungsstelle
befugten Person(en) oder des (der) von ihr ermächtigten Bediensteten:
Datum: 1996-10-18

¹ Angabe des Datums der Ersthinterlegung. Wenn eine erneute Hinterlegung oder eine Weiterleitung vorgenommen worden ist, Angabe des Datums
der jeweils letzten erneuten Hinterlegung oder Weiterleitung.
² In den in Regel 10.2 Buchstabe a Ziffer ii und iii vorgesehenen Fällen Angabe der letzten Lebensfähigkeitsprüfung.
³ Zutreffendes ankreuzen.
⁴ Ausfüllen, wenn die Angaben beantragt worden sind und wenn die Ergebnisse der Prüfung negativ waren.

Formblatt DSMZ-BP/9 (einzige Seite) 0196

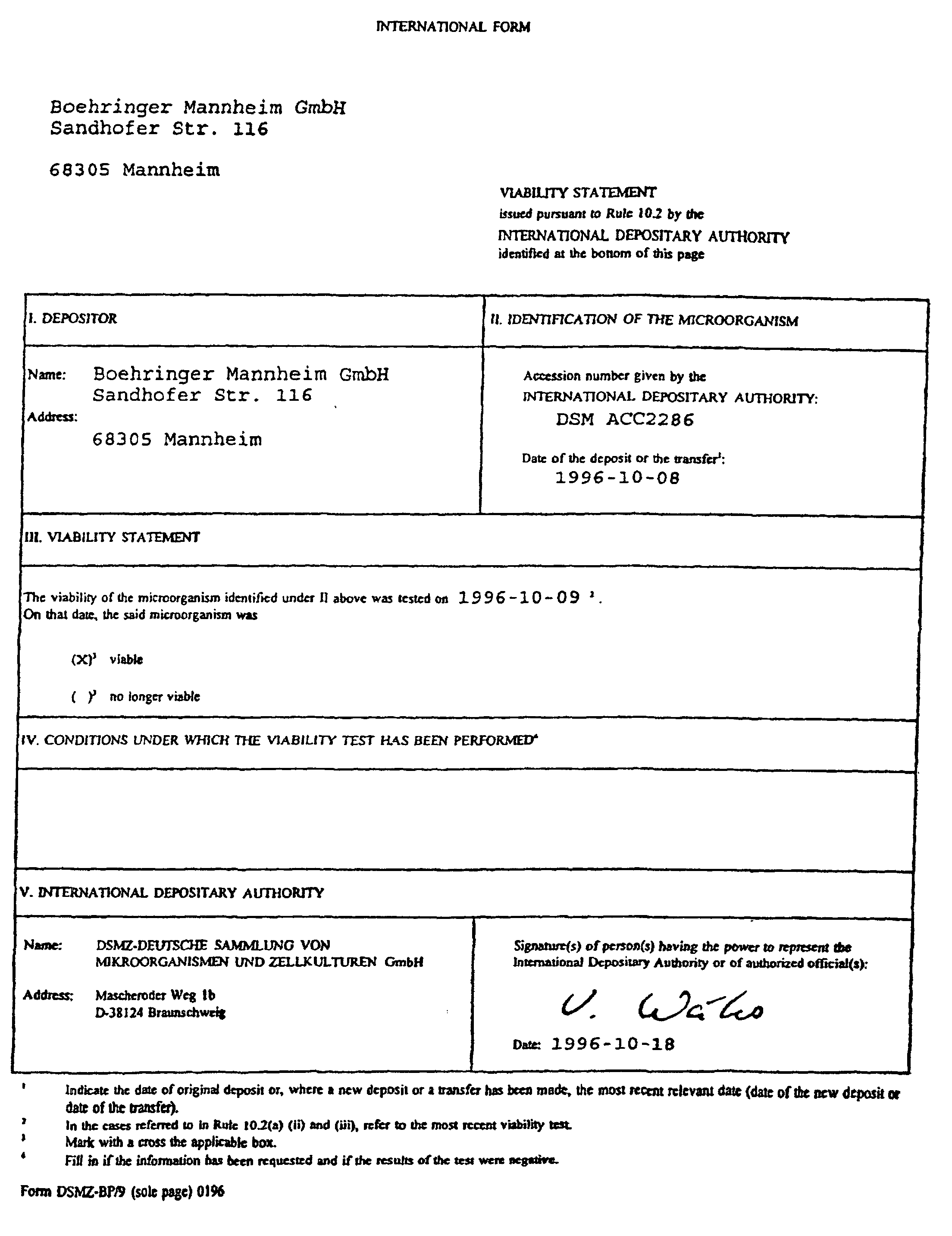
INTERNATIONAL FORM

Boehringer Mannheim GmbH
Sandhofer Str. 116

68305 Mannheim

VIABILITY STATEMENT
issued pursuant to Rule 10.2 by the
INTERNATIONAL DEPOSITARY AUTHORITY
identified at the bottom of this page

I. DEPOSITOR | II. IDENTIFICATION OF THE MICROORGANISM
Name: Boehringer Mannheim GmbH
Sandhofer Str. 116
Address:
68305 Mannheim | Accession number given by the
INTERNATIONAL DEPOSITARY AUTHORITY:
DSM ACC2286
Date of the deposit or the transfer¹:
1996-10-08

III. VIABILITY STATEMENT

The viability of the microorganism identified under II above was tested on 1996-10-09 ².
On that date, the said microorganism was

(X)³ viable

( )³ no longer viable

IV. CONDITIONS UNDER WHICH THE VIABILITY TEST HAS BEEN PERFORMED⁴

V. INTERNATIONAL DEPOSITARY AUTHORITY

Name: DSMZ-DEUTSCHE SAMMLUNG VON
MIKROORGANISMEN UND ZELLKULTUREN GmbH

Address: Mascheroder Weg 1b
D-38124 Braunschweig | Signature(s) of person(s) having the power to represent the
International Depositary Authority or of authorized official(s):
Date: 1996-10-18

¹ Indicate the date of original deposit or, where a new deposit or a transfer has been made, the most recent relevant date (date of the new deposit or date of the transfer).
² In the cases referred to in Rule 10.2(a) (ii) and (iii), refer to the most recent viability test.
³ Mark with a cross the applicable box.
⁴ Fill in if the information has been requested and if the results of the test were negative.

Form DSMZ-BP/9 (sole page) 0196

## Patentansprüche

1. Monoklonale Antiköper gegen das Epitop YPYDVPDYA, das aus dem Hämagglutinin des humanen Influenzavirus abgeleitet ist, bzw. Fragmente hiervon die mindestens 70% der Sequenz YPYDVPDYA entsprechen bzw. um ein bis zwei terminale Aminosäuren verkürzt sind, **dadurch gekennzeichnet, dass** sie eine Affinität von 10⁹ bis 10¹⁰ M⁻¹, ermittelt mittels Plasmonresonanz, besitzen und mittels des HA-Peptids Acetyl-YPYDVPDYAGSGSK (∈-Biotinoyl)-Amid oder Biotinoyl-∈-Aca-SGSGYPYDVPDYA-Amid erhältlich sind.

2. Monoklonale Antikörper nach Anspruch 1, **dadurch gekennzeichnet, dass** sie von Hybridomen produziert werden, die man durch Fusion der Myelomzellen Maus P3x63-Ag8.653 mit B-Lymphozyten aus Lou/C-Ratten erhält, wobei die Lou/C-Ratten mit dem HA-Peptid immunisiert wurden.

3. Monoklonale Antiköper nach Anspruch 2, **dadurch gekennzeichnet, dass** die Immunisierung mit einem an Keyhole Limpet Hämocyanin (KLH) gekoppelten HA-Peptid erfolgt.

4. Monoklonale Antikörper nach einem der Ansprüche 1 bis 3, **dadurch gekennzeichnet, dass** sie vom Hybridom R 3A12, hinterlegt in der Deutschen Sammlung für Mikroorganismen und Zellkulturen unter der Nr. DSM ACC2286 (08.10.1996), produziert werden.

5. Verfahren zur Herstellung von monoklonalen Antikörpern nach einem der Ansprüche 1 bis 4, **dadurch gekennzeichnet, dass** man das HA-Peptid synthetisiert und damit Kleinsäuger immunisiert, die B-Lymphozyten aus der Milz der Tiere gewinnt und mit Myelomzellen Maus P3x62-Ag8.653 fusioniert, die entstandenen Klone, die an ein HA-Peptid und an ein HA-Fusionsprotein binden, selektioniert und von diesen die Klone mit hoher Affinität auswählt und als Hybridzelllinien etabliert.

6. Verfahren nach Anspruch 5, **dadurch gekennzeichnet, dass** man als HA-Peptid Acetyl-YPYDVPDYAGSGSK(ε-Biotinoyl)-Amid oder Biotinoyl-ε-AcaSGSGYPYDVPDYA -Amid einsetzt.

7. Verfahren nach Anspruch 5 oder 6, **dadurch gekennzeichnet, dass** man als HA-Fusionsprotein HA-getaggte Glutathion-S-Transferase einsetzt.

8. Verwendung von monoklonalen Antikörpern nach einem der Ansprüche 1 bis 4, **dadurch gekennzeichnet, dass** sie zum Nachweis und zur Isolierung von nativem Hämagglutinin des humanen Influenzavirus, von modifiziertem Hämagglutinin oder von HA-Fusionsproteinen eingesetzt werden.

## Claims

1. Monoclonal antibodies against the epitope YPYDVPDYA which is derived from the haemagglutinin of the human influenza virus or fragments thereof which correspond to at least 70 % of the sequence YPYDVPDYA or are shortened by one or two terminal amino acids, wherein they have an affinity of 10⁹ to 10¹⁰M⁻¹ determined by means of plasmon resonance and can be obtained by means of the HA peptide acetyl-YPYDVPDYAGSGSK (∈-biotinoyl) amide or biotinoyl-ε-Aca-SGSGYPYDVPDYA amide.

2. Monoclonal antibodies as claimed in claim 1, wherein they are produced by hybridomas which are obtained by fusing mouse P3x63-Ag8.653 myeloma cells with B lymphocytes from Lou/C rats where the Lou/C rats were immunized with the HA peptide.

3. Monoclonal antibodies as claimed in claim 2, wherein the immunization is carried out with a HA peptide coupled to keyhole limpet haemocyanin (KLH).

4. Monoclonal antibodies as claimed in one of the claims 1 to 3, wherein they are produced by the hybridoma R 3A12 deposited at the "Deutsche Sammlung für Mikroorganismen und Zellkulturen" under the No. DSM ACC2286 (08:10.1996).

5. Process for the production of monoclonal antibodies as claimed in one of the claims 1 to 4, wherein the HA peptide is synthesized and used to immunize small mammals, the B lymphocytes are isolated from the spleen of the animals and fused with mouse P3x63-Ag8.653 myeloma cells, the clones that are formed which bind to a HA peptide and to a HA fusion protein are selected and the clones with a high affinity are selected from these and established as hybrid cell lines.

6. Process as claimed in claim 5, wherein acetyl-YPYDVPDYAGSGSK (ε-biotinoyl) amide or biotinoyl-ε-Aca-SGSGYPYDVPDYA amide is used as the HA peptide.

7. Process as claimed in claim 5 or 6, wherein HA-tagged glutathione-S-transferase is used as the HA fusion protein.

8. Use of monoclonal antibodies as claimed in one of the claims 1 to 4, wherein they are used to detect and isolate native haemagglutinin of the human influenza virus, modified haemagglutinin or HA fusion proteins.

## Revendications

1. Anticorps monoclonaux dirigés contre l'épitope YPYDVPDYA qui dérive de l'hémagglutinine du virus humain de l'influenza, respectivement des fragments des premiers cités qui correspondent à la séquence YPYDVPDYA à concurrence d'au moins 70 %, respectivement qui sont raccourcis de 1 à 2 acides aminés terminaux, **caractérisés en ce qu'**ils possèdent une affinité de 10⁹ à 10¹⁰ M⁻¹ déterminée à l'aide d'une résonance des plasmons, et que l'on obtient à l'aide du peptide HA, l'acétyl-YPYDVPDYAGSGSK(ε-biotinyl)-amide ou le biotinyl-ε-Aca-SGSGYPYDVPDYA-amide.

2. Anticorps monoclonaux selon la revendication 1, **caractérisés en ce qu'**ils sont produits à partir d'hybridomes que l'on obtient par fusion des cellules de myélomes de souris P3x63-Ag8.653 avec des lymphocytes B de rats Lou/C, les rats Lou/C ayant été immunisés avec le peptide HA.

3. Anticorps monoclonaux selon la revendication 2, **caractérisés en ce que** l'immunisation a lieu avec un peptide HA couplé à l'hémocyanine de patelle (KLH).

4. Anticorps monoclonaux selon l'une quelconque des revendications 1 à 3, **caractérisés en ce qu'**ils sont produits à partir de l'hybridome R3A12 déposé à la Collection Allemande pour les Micro-organismes et les Cultures cellulaires sous le n° matricule DSM ACC2286 (08.10.1996).

5. Procédé pour la préparation d'anticorps monoclonaux selon l'une quelconque des revendications 1 à 4, **caractérisé en ce qu'**on synthétise le peptide HA et on s'en sert pour immuniser des petits mammifères, on récupère les lymphocytes B de la rate des animaux et on les fusionne avec des cellules de myélomes de souris P3x62-Ag8.653, on sélectionne les clones obtenus qui se lient à un peptide HA et à une protéine de fusion HA et on sélectionne à partir de ces derniers les clones présentant une grande affinité pour les établir à titre de lignée de cellules hybrides.

6. Procédé selon la revendication 5, **caractérisé en ce qu'**on met en oeuvre à titre de peptide HA, l'acétyl-YPYDVPDYAGSGSK(ε-biotinyl)-amide ou le biotinyl-ε-Aca- SGSGYPYDVPDYA-amide.

7. Procédé selon la revendication 5 ou 6, **caractérisé en ce qu'**on met en oeuvre, à titre de protéine de fusion HA, la glutathion-S-transférase marquée par HA.

8. Utilisation d'anticorps monoclonaux selon l'une quelconque des revendications 1 à 4, **caractérisée en ce que** lesdits anticorps sont mis en oeuvre pour le décèlement et pour l'isolation de l'hémagglutinine native du virus humain de l'influenza, de l'hémagglutinine modifiée ou de protéines de fusion HA.
